# EUROPEAN PATENT APPLICATION

(11) **EP 2 960 231 A1**
(43) Date of publication of application: **30.12.2015**
(21) Application number: 14461551.5
(22) Date of filing: 29.06.2014
(51) Int. Cl.: C07C 303/06, C07C 303/08, C07C 309/25, C09K 17/40

(54) **Method of producing a dual ion-exchange resin**

(71) Applicant: 1-szy Dom Spolka z o.o., 41-600 Swietochlowice (PL)
(72) Inventor: Adamczyk, Adam, 51-619 Wroclaw (PL)
(74) Representative: Lampart, Jerzy

(57) **Abstract**

Method of producing a dual ion-exchange resin, characterized by the following steps. In the first stage, the sulfonation of cyclohexanone, chlorosulfonic acid is added in an stoichiometric amount at the temperature of 60-80°C. In the second stage, sulfuric acid and/or oleum from 15% to 20% by weight are added at the temperature of 80 -135° C. The resulting cyclohexanone's sulfonation product is reacted with aromatic compounds containing at least three hydroxyl groups (-OH) in the molecule, in an amount of 1 to 5% by weight. The aromatic compound is preferably quercetin-5'-sulfonic acid.

## Description

The invention relates to the method of production ion-exchange resin applied in construction sector ie building materials production and in the road construction for the ground stabilisation.

Use of ion-exchange resins for the ground stabilisation and recycling, dates back to the sixties of the 20th century. Most of on-exchange stabilising resins applied in the technology are sulfonic connections of different kind, dissolved in strongly concentrated sulphuric acid. Essentially, resins differ in a basic element type, that usually are single associations or their mixtures. The self-ionizing ability of the ion-exchange resin provides permanent hydrogen cation refurbish and anions of the basic element, that can dissolve structural bonds , e.g. of clay and mineral salts found in a soil.

Ion-exchange resin most often consists of oxidant, solvent and the dispersant. An interaction among these elements activates mineral cements naturally occurring in the soil and ties soil molecules altogether creating material showing similarity to most of sedimentary rocks. The resulting material has a higher density and hardness compared with hard rock formations. Filling of internal vacua is encapsulating material what increases its resistance to the water permeation.

Two groups of materials with similar properties are being used in technology.

The first group is protected by US patent No. US4941924 Road stabilizer FR-1 (D-limonene sulfonic acid dissolved in sulphuric acid). The material is produced as liquid ion-exchange concentrated resin with 1.7 g / cm3 density and a pH of 1.0. The material is used for stabilisation as a dilution in water in a ratio of about 1: 200 to 1: 600. The main applicability limitation of this preparation is the presence need of significant quantities of aluminosilicate in the ground mixture.

In order to use the resin EN-1 in sandy soils of low plasticity, it should be enriched with at least 20% admixture of clay, that is a major impediment to the use of this material.

Beside the stabilizer EN-1, similar products using solutions had been developed by the University of Wroclaw. Both resins as EN-1, contain in their basic compositions acids or acid mixtures in concentrated sulphuric acid and detergent. This resin is known from patent application No. PL381672 called Preparation for clay and silty soil stabilization and the process of obtaining clayey and argillaceous ground. The most characteristic feature of the resin is that it is obtained by reaction of a multistage sulphonation in concentrated sulphuric acid and / or fuming sulphuric acid (oleum) derivatives of aromatic hydrocarbons containing acidic groups. Characteristic feature of a process of deriving the preparation for soil stabilization is that the concentrated sulphuric acid and / or fuming sulfuric acid (oleum) is proportionally added in the proper amount of granulated derivatives of aromatic hydrocarbons containing acidic groups, simultaneously vigorously stirring and conducting first reaction stage at a temperature between 50 ° C to 60 ° C for about 6 hours, and then raising the temperature and conducting the second reaction stage at a temperature of 100 ° C to 125 ° C. During reaction conduct, the temperature ought to be controlled strictly. The result of the reaction is a sulfonic derivatives solution in a sulphuric acid solution. Through this method, resins of following compositions are obtained:

### RESINE 1

50% solution of a mixture of acids; 2-hydroxy-naphthalene-6,8-di-sulfonic acid OH-CH₄-(SO₃H)₂, 2-hydroxy-naphthalene-3,6,8-tri-sulfonic acid OH-CH₄-(SO₃H)₃ in 65% sulfuric acid containing 0.5% detergent activesulfonic acid groups. 75% of the acid solution; 3-sulphobenzoic HOOC-C₆H₄-SO₃H, 2-sulphobenzoic HOOC-C₆H₄-SO₃H, 3-sulphobenzoic or 4-sulphobenzoic HOOC -C₆H₄-SO₃H or their mixtures at 65% strength sulfuric acid containing 0.5% detergent with active sulfonic acid groups.

Resins previously used are characterised by a narrow range of application, consist of the necessity ofoccurrence in the ground, the stabilized material, clay content greater than 35%, and simultaneously almost total lack of presence of humus.

All these resins have limited applicability due to the need of large quantity by weight of ground aluminosilicates participation, ie. a minimum of 20-40%.

The study of the applicability of ion-exchange resins understood as a two and three -ring sulphonic compounds having in its molecule sulfonic acid groups of 1 to 3 and a carboxyl or hydroxyl group. It was found that supplementing the basic composition of the ion-exchange resins with water-soluble compounds having hydroxyl groups, eg. synthetic quercetin-5'-sulfonic acid (QSA) with nano-silica and basalt flour and cement or fly ash in the proper quantities to the composition of the clay in the soil, results that the required clay content can be reduced to an amount of approx. 15%. Achieving the pursued effect is possible when applied as a primary ingredient of mono-, di-, trisulfonic cyclohexanone acids.

The base of the invention is the method for producing dual ion-exchange resin, consisting of sulphonated at elevated temperature substituent as hydrocarbon compounds and the addition of concentrated sulphuric acid and / or fuming sulphuric acid - oleum. In the first stage of the cyclohexanone sulphonation, chlorosulphonic acid is used in stoichiometric amounts at the temperature of 60-80 C degrees. In the second stage, sulphuric acid and/or fuming sulphuric acid are being added from the 15% to the 20% by weight, in the temperature of 80 C degrees to the temperature of 135 C degrees and next, product of cyclohexanone sulphonating is being linked with aromatic compounds, containing at least 3 groups (- OH) in the particle, favourably quercetin-5-sulfonic acid in the amount from 1 up to the 5% by weight.

The resin significantly increases its applicability particularly in soils with low content of aluminosilicates, and expands the area of possible applications to a group of construction materials.

### Example 1

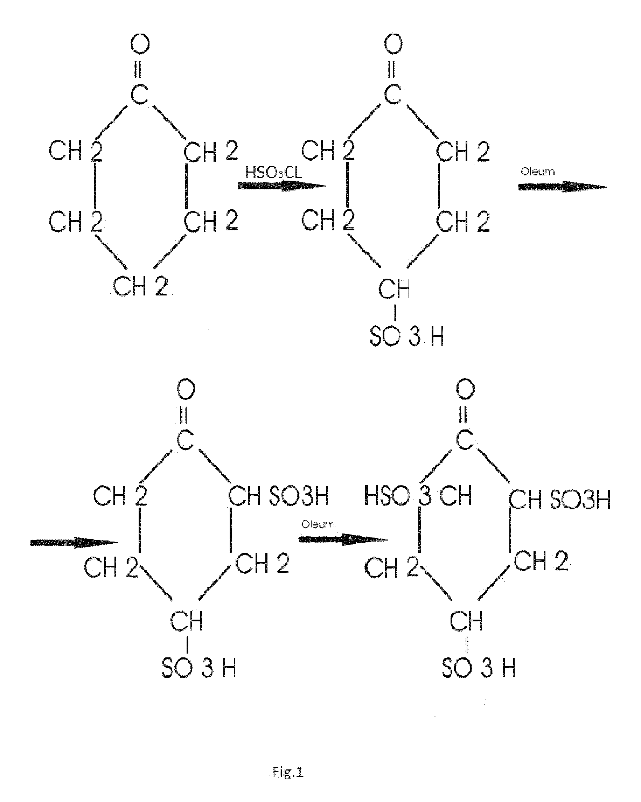

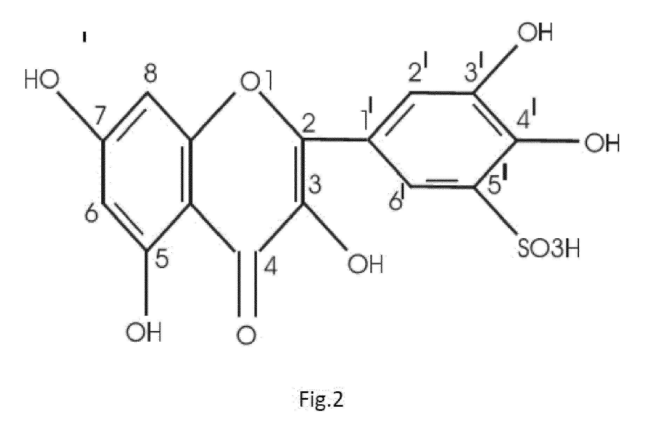

Reactions of producing ion-exchange resins take place as in fig.1, however fig.2 presents the chemical formula of quercetin-5-sulfonic acid. In the first stage, of the sulphonation of 98 kg of cyclohexanone and 116 kg of chlorosulphonic acid are used and joined at the temperature of 60 ° C. In the second step, sulphuric acid in an amount of 196 kg or oleumin an amount of 234 kg at the temperature of 80 ° C are added. Then, the cyclohexanone sulphonation product is combined with an quercetin-5-sulfonic acid in an amount of 3.86 kg. As a result of the reaction, a cyclohexanone mono-, di- and trisulphonic acid solutions in sulphuric acid are successively formed.

### Example 2

In the first stage, of the sulphonation 98 kg of cyclohexanone and 116 kg of chlorosulphonic acid is used and joined at the temperature of 80 ° C. In the second step, sulphuric acid in an amount of 196 kg or oleum in an amount of 234 kg in temperature of 135 ° C is added. Then, the cyclohexanone sulphonation product is combined with an quercetin-5-sulfonic acid in an amount of 19.25 kg.

The preparation is characterised by the following parameters:
- The specific weight of 1.78 g / cm3- pH 1-1.5
- Preparation of a greenish-brown colour, designed to be diluted with water in a ratio of 1: 150 to 1: 250. The resulting product in aqueous solution is completely environment-friendly while after mixing with the soil creates a material similar to natural.

The technological manufacturing process begins with accurate measurement and weighing substrates that are cyclohexanone, chlorosulfonic acid, sulphuric acid, oleum and the quercetin-5'-sulfonic acid. The reactor according to the program, is filled the basic constituent, and heating and mixing in the reactor's contents starts. After implementing all substrates, a heating process with simultaneous mixing up starts. After obtaining final temperature, the mixing process proceeds another few hours accordingly to the program. For about 1 hour before the scheduled process end, a test measuring the degree of the reaction is performed and depending on the result, the further process is proceed accordingly to the program or it is corrected. After process completion, the product is cooled and is poured into commercial packaging.

The overall technological process requiring control and supervision takes about 8 hours.

## Claims

1. Method of producing dual ion-exchange resin, consists in sulphonation process at the elevated temperature of substituent as hydrocarbon compounds and addition of concentrated sulphuric acid and/or fuming sulphuric acid - oleum, **characterized in that** it performs the following steps, the first stage of the sulphonation of cyclohexanone is use of chlorosulfonic acid in stoichiometric amounts at a temperature from 60 to 80 degrees ° C while in the second stage the sulphuric acid and / or oleum from 15% to 20% by weight in temperature from 80 to 135 degrees ° C, and then cyclohexanone sulphonation product connects with aromatic compounds containing at least 3 group (-OH) in the molecule, preferably quercetin-5'-sulphonic acid in an amount of from 1 to 5% by weight.
